# EUROPÄISCHE PATENTSCHRIFT

(11) **EP 0 361 486 B1**
(45) Veröffentlichungstag und Bekanntmachung des Hinweises auf die Patenterteilung: **03.03.1993**
(21) Anmeldenummer: 89117996.2
(22) Anmeldetag: 28.09.1989
(51) Int. Cl.: C07C 211/52, C07C 209/00, C07C 205/58, C07C 229/52, C07C 237/30, C07C 233/65, C07C 245/08, C08G 69/26

(54) **Fluorhaltige Monomere, Verfahren zu ihrer Herstellung und ihre Verwendung**
Fluorinated monomers, process for their preparation and their use
Monomères fluorés, procédé pour leur préparation et leur utilisation

(30) Priorität: 30.09.1988 DE 3833338
(43) Veröffentlichungstag der Anmeldung: 04.04.1990
(73) Patentinhaber: HOECHST AKTIENGESELLSCHAFT, 65926 Frankfurt am Main (DE)
(72) Erfinder: Lau, Jürgen, Dr., D-6238 Hofheim am Taunus (DE); Siegemund, Günter, Dr., D-6238 Hofheim am Taunus (DE); Röhrscheid, Freimund, Dr., D-6233 Kelkheim(Taunus) (DE)

(56) Entgegenhaltungen:
- US-A- 3 310 573
- US-A- 4 645 824
- CHEMICAL ABSTRACTS, Band 97, Nr. 22, 29. November 1982, Columbus, Ohio, USA; K. S. Y. LAU et al, "Synthesis of polymer intermediates containing the hexafluoroisopropylidene group via functionalization of 2,2-diphenylhexafluoropropane" Seite 1, Zusammenfassung Nr. 182 895d

## Beschreibung

Die Erfindung betrifft fluorhaltige Monomere und ihre Herstellung, insbesondere 2-(3-Aminophenyl)-2-(4-aminophenyl)-hexafluorpropan, einem neuen fluorhaltigen Monomer zur Herstellung von hochtemperaturbeständigen Polykondensaten wie Polyamide und Polyimide.

Es ist bekannt, daß aus 2,2-Bis(3-aminophenyl)-hexafluorpropan und 2,2-Bis(4-aminophenyl)hexafluorpropan, hochtemperaturstabile und transparente Polymere hergestellt werden können (US-PS 3.356.648, DE-OS 3.526.010, US-PS 4.645.824). Aufgrund obiger Eigenschaften werden diese Polymere als hochwertige Werkstoffe z.B. in der Luftfahrt- und Elektronikindustrie verwendet.

Die Aufgabe bestand darin, ein teilfluoriertes Diamin herzustellen, mit dem Polykondensate erhalten werden können, die die Eigenschaften der Polykondensate auf der Basis der obengenannten strukturisomeren Diaminoverbindungen in sich vereinigen.

Gegenstand der Erfindung sind neue Verbindungen der Formel 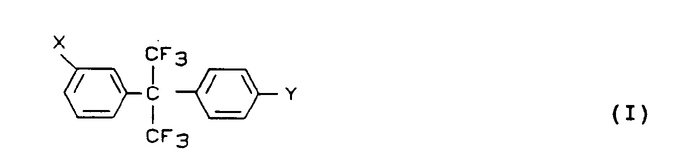
mit,
X = Wasserstoff, NO₂, NH₂ und
Y = CH₃, COOH, CONH₂, NH₂, -N=N-Z, wobei Z die Gruppe 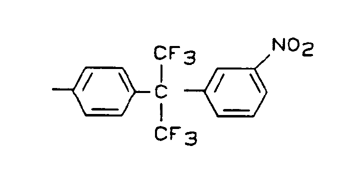
ist,
mit der Maßgabe, daß, wenn Y die Gruppe -N=N-Z darstellt, X nur NO₂ ist und wenn Y die Gruppe -CH₃ darstellt, X nur Wasserstoff ist, vorzugsweise 2-(3-Aminophenyl)-2-(4-aminophenyl)-hexafluorpropan (II) sowie Verfahren zu ihrer Herstellung und ihre Verwendung.

Ausgehend von 2-(4-Methylphenyl)-hexafluorpropanol-2 oder 2-Phenyl-hexafluorpropanol-2 wird die Verbindung (II) über sechs Reaktionsschritte erhalten. Die dabei gebildeten Zwischenprodukte sind ebenfalls neu.

Die Methylverbindung (III) kann nach zwei Verfahren hergestellt werden, und zwar a) durch Kondensation von einem Mol 2-(4-Methylphenyl)-hexafluorpropanol-2 mit einem Mol Benzol oder b) durch Kondensation von einem Mol 2-Phenylhexafluorpropanol-2 mit einem Mol Toluol in Gegenwart von Fluorwasserstoff. Die Ausgangsverbindungen sind bekannt und in J.O.C., 998-1001, 30 (1965) beschrieben. Die Reaktionstemperaturen bei den Verfahren a) und b) liegen zwischen 80 und 180°C, vorzugsweise zwischen 100 und 170°C. Die Reaktionszeiten betragen 24 bis 90, vorzugsweise 65 bis 90 Stunden. Das Molverhältnis der eingesetzten Reaktionspartner beträgt in beiden Fällen im allgemeinen 1:5, vorzugsweise 1:1,5 bis 2,5, wobei die Verbindungen Benzol bzw. Toluol stets die Überschußkomponenten sind. Der Anteil an Fluorwasserstoff, der bei der Reaktion zur Herstellung der Verbindung (III) nötig ist, wird auf die entsprechende Ausgangsverbindung bezogen und liegt bei beiden Verfahren im allgemeinen im Molverhältnis 1:7 bis 25, vorzugsweise 1:9 bis 20. Zur Aufarbeitung des Reaktionsgutes wird im allgemeinen der Fluorwasserstoff nach Beendigung der Reaktion bei ca. 80°C aus dem Reaktor abgegast.
Das erhaltene Rohgemisch wird mit Wasser gewaschen, getrocknet und fraktioniert destilliert.

Die Methylverbindung (III) kann nach den üblichen stöchiometrischen Methoden mit z.B. Kaliumpermanganat, Chromsäure/Eisessig, Dichromat/Schwefelsäure oder bevorzugt katalytisch mit molekularem Sauerstoff in Gegenwart einer Katalysatorkombination aus den Ionen von Kobalt, Mangan und Brom zu der Carbonsäure (IV) oxidiert werden, wobei in einem sauren Medium gearbeitet wird, das zumindestens 40 % aus Essigsäure oder Propionsäure oder deren Mischungen besteht. Essigsäure ist wegen ihrer größeren Beständigkeit gegen einen oxidativen Abbau vorzuziehen. Das Verhältnis von saurem Medium zu eingesetzter Ausgangssubstanz kann bis zu einem Verhältnis von 40:60 Gew.-%, bezogen auf die Gesamtreaktionsmasse, angewandt werden.

Bromidionen sind für den vollständigen Ablauf der Oxidation unbedingt notwendig. Kobalt- und Manganionen werden im allgemeinen im Verhältnis von 3:1 bis 1:3, vorzugsweise 1:1 eingesetzt. Die Summe der Konzentrationen der beiden Elemente beträgt im allgemeinen 0,01 bis 0,2, vorzugsweise 0,02 bis 0,12 und insbesondere 0,04 bis 0,08 g-Atom/kg Gesamtmasse. Das Verhältnis der Summe von Kobalt und Mangan zu Brom ist im allgemeinen 1:0,01 - 2, vorzugsweise 1:0,025-1 und insbesondere 1:0,05-0,2. Es ist auch möglich, zusätzlich zu den beiden Metallionen des Katalysators auch Cerionen einzusetzen. Diese katalysieren die Oxidation unvollständig oxidierter Zwischenstufen. Ihre Anwesenheit erhöht die Reinheit und die Ausbeute der teilfluorierten Monocarbonsäure. Die Cerionen werden dem Katalysator im Verhältnis der Summe der Kobalt- und Manganionen zu Cerionen wie 1:0,02-2, vorzugsweise 1:0,05-1 und insbesondere 1:0,2-0,6 zugefügt. Vorzugsweise werden die Metallionen in Form ihrer Acetate eingesetzt.

Brom kann in Form von Bromiden z.B. der Bromide der Alkalimetalle, einschließlich dem Ammoniumbromid, und denen der Metalle Kobalt, Mangan und Cer oder als Lösung von Bromwasserstoff in Wasser oder Eisessig eingesetzt werden. Daneben können auch bromhaltige organische Verbindungen verwendet werden, die während der Oxidation zerfallen und Bromionen freisetzen z.B. Tetrabrommethan.

Die Oxidation wird im allgemeinen bei Temperaturen von 120 bis 220, vorzugsweise 140 bis 190 und insbesondere 155 bis 180°C durchgeführt. Der Druck im Reaktor liegt im allgemeinen zwischen 5 und 40, vorzugsweise zwischen 10 bis 30 und insbesondere zwischen 14 bis 20 bar.

Für die Verfahrensweise ist es günstig, daß die zur Oxidation benötigte Luft nahe dem Boden des Reaktors in die Flüssigphase eingeleitet und durch starkes Rühren oder durch spezielle Düsen in der Flüssigphase fein verteilt wird.

Im dritten Reaktionsschritt wird durch eine Nitrierungsreaktion aus der Carbonsäure (IV) das 2-(4-Carboxyphenyl)-2-(3-nitrophenyl)hexafluorpropan (V) hergestellt. Für die Nitrierung können allgemein übliche Nitriersäuregemische z.B. Salpetersäure/Schwefelsäure, Salpetersäure/Eisessig, Salpetersäure/Essigsäureanhydrid oder Salpetersäure/Wasser-Gemische benutzt werden. Insbesondere eignet sich 98 % Salpetersäure zur Nitrierung, wobei die Carbonsäure in einem organischen Lösungsmittel, z.B. Tetrahydrofuran, Dioxan, Eisessig, Äthanol, halogenierte aliphatische Kohlenwasserstoffe mit bis zu 3 C-Atomen, vorzugsweise Dichlormethan, und konzentrierte Schwefelsäure, gelöst wird. Die konzentrierte Salpetersäure wird anschließend bei einer Temperatur von minus 20 bis plus 50°C, vorzugsweise bei minus 5 bis plus 5°C, jeweils in kleinen Mengen zugegeben.
Die Verbindung (V) wird anschließend in das 2-(4- Carbonsäureamidophenyl)-2-(3-nitrophenyl)-hexafluorpropan (VI) überführt. Für die Herstellung des Carbonsäureamids (VI) können die allgemein üblichen Methoden zur Darstellung von Carbonsäureamiden aus Carbonsäuren angewandt werden. Insbesondere eignet sich die direkte Umsetzung der Carbonsäure (V) mit Amidosulfonsäure in einem Reaktionsgemisch aus Schwefelsäure/Oleum bei einer Temperatur von 50 bis 180°C, vorzugsweise von 90 bis 100°C.

Im fünften Reaktionsschritt wird die Carbonsäureamidoverbindung (VI) unter den Bedingungen der "Hofmann"-Umlagerung mit wäßrigen Hypohalogenid-Lösungen in Gegenwart von Basen, bei Reaktionstemperaturen von 5 bis 70°C, vorzugsweise ca. 40°C, umgesetzt. Überraschenderweise wird hierbei das erwartete 2-(4-Aminophenyl)-2-(3-nitrophenyl)hexafluorpropan (VIIa) nur in untergeordneten Mengen erhalten. Der überwiegende Anteil des Reaktionsproduktes stellt das 4,4′-Bis[2-(3-nitrophenyl)hexafluorpropan]azobenzol (VII) dar. Als Hypohalogenid-Lösungen werden 5 bis 30 %ige wäßrige Lösungen aus Alkalihypochloriden und -hypobromiden, vorzugsweise eine ca. 13 %ige Natriumhypochlorid-Lösung, in Gegenwart von 5-50 Gew.-% einer Base, z.B. Alkalihydroxid oder Ammoniumbasen, eingesetzt. Zur Reaktionsbeschleunigung können Phasentransferkatalysatoren, z.B. Tetraalkylammoniumsalze, Benzyltrialkylammoniumsalze, Benzyltrialkylphosphoniumsalze, Benzyltriphenylphosphoniumsalze, Tetraalkylphosphoniumsalze mit jeweils 1-6°C-Atomen im Alkylrest, Kronenäther oder Polyäthylenglykole, in Mengen von 0,1 bis 20 mol % zugesetzt werden.

In dem fünften Reaktionsschritt wird neben der Azoverbindung (VII), die zu etwa 70 % im Rohprodukt enthalten ist, in einem geringen Umfang - d.h. bis zu 15 % - 2-(4-Aminophenyl)-2-(3-nitrophenyl)hexafluorpropan (VIIa) als weiteres Produkt gebildet. Die reine Azoverbindung (VII) kann durch Umkristallisation des Rohproduktes aus einem organischen Lösungsmittel, z.B. Äthanol, Acetonitril, Essigsäureäthylester, Dichlormethan, Chloroform, Tetrachlorkohlenstoff, vorzugsweise Äthanol, erhalten werden. Für die Herstellung des 2-(3-Aminophenyl)-2-(4-aminophenyl)hexafluorpropans (II) ist die Isolierung der Azoverbindung (VII) aus dem Rohprodukt allerdings nicht notwendig, da sowohl aus der Verbindung (VII) als auch aus (VIIa) die Verbindung (II) erhalten wird. Die Reduktion der Nitro- und der Azogruppe in den Verbindungen (V), (VI), (VII) und (VIIa) kann z.B. nach den üblichen katalytischen Methoden mit Hydrierkatalysatoren eines Übergangsmetalls, insbesondere der VIII. Nebengruppe des Periodischen Systems oder nach stöchiometrischen Methoden (z.B. mit Zinn(II)-chlorid/Eisessig) durchgeführt werden. Für die katalytischen Reduktionen können z.B. Platinmetalle, Kupfer, Eisen, Kobalt, Nickel, Metalloxide oder gemischte Metallkatalysatoren, vorzugsweise ohne oder mit Anwendung von Überdruck in üblichen Verdünnungsmitteln wie niedrigen aliphatischen Alkoholen, aromatischen Kohlenwasserstoffen (z.B. Toluol, Xylol), aliphatischen Monocarbonsäure-Estern oder ähnlichen organischen Lösungsmitteln bei Temperaturen von z.B. 10-130°C eingesetzt werden. Für die Reinigung der Verbindung (II) kann diese bei z.B. 10-100°C mit Säuren in eines ihrer wasserlöslichen Salze (z.B. Halogenid, Hydrogensulfat) übergeführt werden, das gegenüber Aminogruppen unter den angewandten Bedingungen inert ist.

Die erhaltene 3,4′-Diaminoverbindung eignet sich zur Herstellung von hochtemperaturbeständigen Polykondensaten. Durch Umsetzung mit Tetracarbonsäuren oder deren Derivaten werden Polyimide erhalten. Andererseits ergibt die Reaktion mit Dicarbonsäurechloriden Polyamide. Beispielsweise weisen die Polyimide niedrige Dielektrizitätskonstanten auf.

Weiterhin können neue Monomere und Oligomere erhalten werden, beispielsweise durch Reaktion mit Dianhydriden. Die erhaltenen Imidmonomeren und Oligomeren können durch Additionsreaktionen gehärtet werden. Das erfindungsgemäße Diamin ist weiterhin geeignet zur Herstellung von polymeren Vorprodukten, Epoxidhärtern, Matrixharzen, Laminaten, Filmen, Fasern, Klebstoffen, Beschichtungen, Fotoresists und Formkörpern.

### Beispiele

### 1) 2-(4-Methylphenyl)-2-phenyl-hexafluorpropan (III)

a) In einem 5 l Stahlautoklaven wurden 1290 g 2-(4-Methylphenyl)-hexafluorpropanol-2 und 780 g Benzol vorgelegt und 1500 g wasserfreier Fluorwasserstoff in den geschlossenen Autoklaven eingepumpt. Das Reaktionsgemisch wurde unter Rühren für 64 Stunden auf 170-175°C erhitzt. Nach Ende der Reaktion wurde Fluorwasserstoff bei 80°C abgegast, anschließend das flüssige Produkt 2 x mit Wasser gewaschen, über Calciumchlorid getrocknet und fraktioniert destilliert. Siedepunkt 135-136°C/1,4 mbar. Auswaage: 1424 g (89,5 % der Theorie).
   - C₁₆H₁₂F₆: ber.: C 60,38 % H 3,77 % F 35,85 %
   - MG: 318: gef.: C 60,50 % H 3,70 % F 35,80 %
b) In einem 1 l V4A-Stahlautoklaven mit Rührer wurden 244 g 2-Phenylhexafluorpropanol-2, 368 g Toluol und 180 g wasserfreier Fluorwasserstoff vereinigt. Das Reaktionsgemisch wurde anschließend 65 Stunden bei 175°C gerührt. Nach Abkühlen auf 80°C wurde der Fluorwasserstoff abgegast. Das Rohprodukt wurde 2 x mit Wasser gewaschen, über Calciumchlorid getrocknet und fraktioniert destilliert. Siedepunkt 82°C/0,05 mbar. 67 g =̂ 21 % der Theorie.

### 2) 2-(4-Carboxyphenyl)-2-phenyl-hexafluorpropan (IV)

In einem 1 l-Glasautoklaven wurden 298 g 2-(4-Methylphenyl)-2-phenyl-hexafluorpropan, 2,49 g Kobalt(II)acetattetrahydrat, 2,45 g Mangan(IV)acetattetrahydrat, 0,41 g Bromwasserstoff (entsprechend 4,1 g einer 10 %igen HBr-Lösung in Eisessig) vorgelegt. Unter 6,5 bar Sauerstoffdruck wurde die Mischung unter exothermer Reaktion bis ca. 180°C erhitzt und 1 Stunde bei 170-180°C befassen. Aus der auf ca. 100°C abgekühlten Reaktionslösung wurde anschließend 200 g Essigsäure abdestilliert. Die im Kolben verbleibende Lösung (ca. 600 g) wurde in der Siedehitze langsam mit 275 g Wasser versetzt. Die auskristallisierte Carbonsäure wurde abgesaugt und zweimal mit je 75 ml 50 %iger wäßriger Essigsäure und fünfmal mit je 85 ml Wasser gewaschen und bei 60°C/60 mbar getrocknet. Ausbeute: 311 g (95,5 % d.Th.)
Fp.: 153-155°C farblose kleine Kristalle
- C₁₆H₁₀F₆O₂: ber.: C 55,18 H 2,89 F 32,74 O 9,19
- (348): gef.: C 55,20 H 3,00 F 33,20 O 9,00

### 3) 2-(4-Carboxyphenyl)-2-(3-nitrophenyl)hexafluorpropan (V)

261 g 2-(4-Carboxyphenyl)-2-phenylhexafluorpropan wurden in 500 ml Methylenchlorid suspendiert und nach der Zugabe von 188 ml konzentrierter Schwefelsäure bei minus 5 bis 0°C tropfenweise mit 75 ml konzentrierter Salpetersäure versetzt. Die Reaktionsmischung wurde bei dieser Temperatur 1 Stunde nachgerührt und anschließend auf 2000 g Eis gegossen. Der Feststoff wurde abfiltriert und mit Wasser gewaschen, bis das Waschwasser einen pH-Wert von 3-4 aufwies. Rohprodukt 208 g, Fp. 180-185°C.
Aufarbeitung des Filtrates: Die organische Phase wurde abgetrennt, 2 mal mit Wasser gewaschen, über Magnesiumsulfat getrocknet und eingeengt. Der klebrige, gelbe Rückstand wurde zweimal aus Toluol umkristallisiert, wonach weitere 30 g Rohprodukt mit einem Schmelzpunkt von 180-184°C erhalten wurden.
Die vereinigten Rohproduktmengen (238 g) wurden zweimal aus Toluol umkristallisiert, wonach 186 g (63 % d.Th.) eines weißen Feststoffes erhalten wurden, der eine gaschromatographisch bestimmte Reinheit von 99,2 % besaß.
¹H-NMR (CDCl₃) δ (ppm): 7,5-7,7 m 4H, 8,1-8,4 m 4H, 11,1 s 1H
¹⁹F-NMR (CDCl₃) δ (ppm): -64,0 s
IR (KBr-Preßling): 1170-1280 cm⁻¹ CF₃, 1350 und 1540 cm⁻¹ NO₂, 1700 cm⁻¹ C = O, 2500-3450 cm⁻¹ OH
- C₁₆H₉F₆NO₄: ber.: C 48,87 H 2,31 N 3,56 F 28,99 O 16,28
- (393,23): gef.: C 49,20 H 2,00 N 3,40 F 28,70 O 16,50

### 4) 2-(4-Carbonsäureamidophenyl)-2-(3-nitrophenyl)hexafluorpropan (VI)

198 g 2-(4-Carbonsäurephenyl)-2-(3-nitrophenyl)hexafluorpropan wurden in eine Mischung aus 700 ml konzentrierter Schwefelsäure und 350 ml Oleum (65 %ig) eingetragen. Nach der Zugabe von 200 g Amidosulfonsäure wurde die Reaktionsmischung 3 Stunden bei 90-95°C erhitzt. Die auf etwa 20°C abgekühlte Suspension wurde unter ständigem Rühren auf ca. 6 kg Eis gegossen. Anschließend wurde der ausgefallene Feststoff abfiltriert und mit Wasser neutral gewaschen.
Ausbeute: 191 g (97 % d.Th.) weißer Feststoff
Fp.: 147-148°C
¹H-NMR (CDCl₃) δ (ppm): 6,6 breites s 2H, 7,4-7,9 m 6H, 8,3-8,4 m 2H
¹⁹F-NMR (CDCl₃) δ (ppm): -64,1 s
IR (KBr-Preßling): 1140-1260 cm⁻¹ CF₃, 1620 und 1660 cm⁻¹ CONH₂, 3200 und 3400 cm⁻¹ NH₂
- C₁₆H₁₀F₆N₂O₃: ber.: C 48,98 H 2,57 N 7,14
- (392,26): gef.: C 49,40 H 2,60 N 7,20

### 5) 4,4′-Bis[2-(3-nitrophenyl)hexafluorisopropyl]azobenzol (VII)

157 g 2-(4-Carbonsäureamidophenyl)-2-(3-nitrophenyl)hexafluorpropan wurden bei 0 bis 5°C in eine Mischung aus 900 ml 13 %iger wäßriger Natriumhypochloridlösung, 150 ml 50 %iger Natriumhydroxid-Lösung und 5 ml Tricaprylylmethylammoniumchlorid eingetragen. Die Suspension wurde 24 Stunden gerührt, wobei die Reaktionstemperatur nicht 50°C übersteigen sollte. Die Reaktionsmischung wurde mit verdünnter Essigsäure neutralisiert, der Feststoff abfiltriert und mit Wasser gewaschen. Nach der Umkristallisation des getrockneten Rohproduktes (149 g) wurden 61 g eines Feststoffes erhalten mit einem Schmelzpunkt von 185-187°C. Die Aufarbeitung der Mutterlauge ergab weitere 14 g des Produktes.
Ausbeute: 75 g (52 % d.Th.)
¹H-NMR (CDCl₃) δ (ppm): 7,5-8,0 m 12H, 8,3-8,4 m 4H
¹⁹F-NMR (CDCl₃) δ (ppm): -64,1 s
IR (KBr-Preßling): 1140-1260 cm⁻¹ CF₃, 1350 und 1540 cm⁻¹ NO₂
- C₃₀H₁₆F₁₂N₄O₄: ber.: C 49,73 H 2,23 N 7,73
- (724,46): gef.: C 49,60 H 2,40 N 7,50

### 6) 2-(3-Aminophenyl)-2-(4-aminophenyl)hexafluorpropan (II)

74,2 g 4,4′-Bis[2-(3-nitrophenyl)hexafluorisopropyl]azobenzol wurden in 600 ml Essigsäureäthylester gelöst und in einem Autoklaven nach der Zugabe von 1 g Pd/C 5 %ig zunächst bei 25°C und bei Nachlassen der Reaktion bei 100°C mit Wasserstoff (100 bar) reduziert. Nach dem Abfiltrieren des Katalysators wurde das Lösungsmittel abgetrennt. Der Rückstand wurde mit verdünnter Salzsäure aufgenommen und mit Aktivkohle behandelt. Das daraus erhaltene farblose Filtrat wurde mit halbkonzentrierter Ammoniak-Lösung neutralisiert. Der dabei ausgefallene Niederschlag wurde abfiltriert, mit Wasser gewaschen und bis zur Gewichtskonstanz getrocknet.
Ausbeute: 48 g (72 % d.Th.) weißer Feststoff
Fp.: 142-143°C
¹H-NMR (CDCl₃) δ (ppm): 3,7 breites s 4H, 6,6-6,9 m 5H, 7,0-7,2 m 3H
¹⁹F-NMR (CDCl₃) δ (ppm): -64,3 s
- C₁₅H₁₂F₆N₂: ber.: C 53,89 H 3,62 F 34,10 N 8,38
- (334,27): gef.: C 53,50 H 3,50 F 33,90 N 8,20

### 7) 2-(3-Aminophenyl)-2-(4-carboxyphenyl)hexafluorpropan bzw.

2-(3-Aminophenyl)-2-(4-Carbonsäureamidophenyl)hexafluorpropan

Aus 2-(4-Carboxyphenyl)-2-(3-nitrophenyl)hexafluorpropan (V) bzw. 2-(4-Carbonsäureamidophenyl)-2-(3-Nitrophenyl)hexafluorpropan (VI) wurden in analoger Verfahrensführung wie in Beispiel 6 die entsprechenden Aminophenylverbindungen erhalten.

## Patentansprüche

1. Verbindungen der Formel 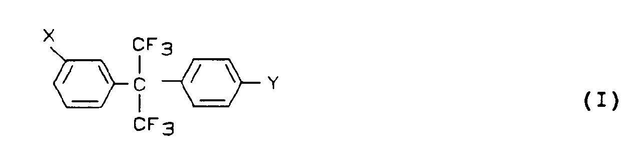 mit,
X = Wasserstoff, NO₂, NH₂ und
Y = CH₃, COOH, CONH₂, NH₂, -N=N-Z, wobei die Gruppe Z 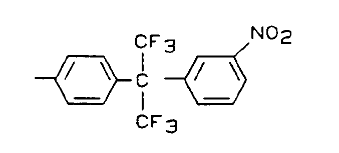 ist
mit der Maßgabe, daß, wenn Y die Gruppe -N=N-Z darstellt, X nur NO₂ ist und wenn Y die Gruppe -CH₃ darstellt, X nur Wasserstoff ist.

2. Verbindung der Formel

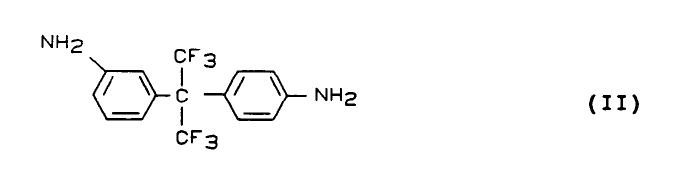

3. Verfahren zur Herstellung der Verbindung der Formel II in einem mehrstufigen Prozeß, dadurch gekennzeichnet, daß
1a) 2-(4-Methylphenyl)hexafluorpropanol-2 mit Benzol oder
1b) 2-Phenylhexafluorpropanol-2 mit Toluol in Gegenwart von Fluorwasserstoff kondensiert wird,
2) Das erhaltene 2-(4-Methylphenyl)-2-phenylhexafluorpropan (III) zum entsprechenden Carbonsäurederivat (IV) oxidiert wird,
3) aus (IV) durch Nitrierung 2-(4-Carboxyphenyl)-2-(3-nitrophenyl)hexafluorpropan (V) erhalten wird, das dann
4) durch Umsetzung mit Amidosulfonsäure in 2-(4-Carbonsäureamidophenyl)-2-(3-nitrophenyl)hexafluorpropan (VI) übergeführt wird,
5) aus (VI) durch Umsetzung mit Alkalihypohalogenidlösung im alkalischen Medium 4,4′-Bis[2-(3-nitrophenyl)hexafluorisopropyl]azobenzol (VII) gebildet wird, das dann
6) zu 2-(3-Aminophenyl)-2-(4-aminophenyl)hexafluorpropan (II) reduziert wird.

4. Verfahren nach Anspruch 3, dadurch gekennzeichnet, daß in Stufe 5) neben der Verbindung (VII) in untergeordneter Menge 2-(3-Nitrophenyl)-2-(4-aminophenyl)-hexafluorpropan (VIIa) gebildet wird.

5. Verfahren nach Anspruch 3, dadurch gekennzeichnet, daß die Reduktion in Stufe 6) katalytisch mit Hydrierkatalysatoren eines Übergangsmetalls des periodischen Systems, insbesondere mindestens einem Metall der VIII. Nebengruppe oder nach stöchiometrischen Methoden durchgeführt wird.

6. Verfahren nach einem oder mehreren der Ansprüche 3 bis 5, dadurch gekennzeichnet, daß die Verbindungen (V), (VI) und (VIIa) gemäß Stufe 6) zu den entsprechenden Aminoverbindungen reduziert werden.

7. Verfahren nach Anspruch 3, dadurch gekennzeichnet, daß die Oxidation der Verbindung (III) durch Einleiten von Luftsauerstoff in ein saures organisches Medium bei Temperaturen von 120 bis 220°C und einem Druck zwischen 5 und 40 bar in Gegenwart eines Gemisches von Verbindungen der Metalle Co und Mn sowie von Bromidionen vorgenommen wird.

8. Verfahren nach Anspruch 7, dadurch gekennzeichnet, daß das Verhältnis von Kobalt zu Mangan gleich 3:1 bis 1:3, vorzugsweise 1:1 ist, wobei die Summe der Konzentrationen der beiden Elemente Kobalt und Mangan gleich 0,01-0,20 g-Atom/kg Gesamtreaktionsmasse, vorzugsweise 0,02-0,12, insbesondere 0,04-0,08 g-Atom/kg beträgt.

9. Verfahren nach Anspruch 7 oder 8, dadurch gekennzeichnet, das als zusätzliches Metallion Cer im Katalysator enthalten ist im Verhältnis von der Summe von Kobalt und Mangan zu Cer wie 1:0,02-2, vorzugsweise 1:0,05-1, insbesondere 1:0,2-0,6.

10. Verfahren nach einem oder mehreren der Ansprüche 7 bis 9, dadurch gekennzeichnet, daß die Metallionen in Form von Acetatverbindungen zugesetzt werden.

11. Verfahren nach einem oder mehreren der Ansprüche 7 bis 10, dadurch gekennzeichnet, daß Brom in Form von Bromiden oder als Lösung von Bromwasserstoff in Wasser oder Eisessig eingesetzt wird.

12. Verwendung der Verbindung gemäß Anspruch 2 zur Herstellung von linearen Polycarbonsäureamiden und -imiden.

13. Verwendung nach Anspruch 12, dadurch gekennzeichnet, daß Formkörper, Filme und Fasern hergestellt werden.

## Claims

1. A compound of the formula 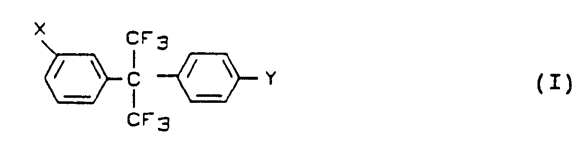 where
X = hydrogen, NO₂ or NH₂ and
Y = CH₃, COOH, CONH₂, NH₂ or -N=N-Z, in which Z is the group 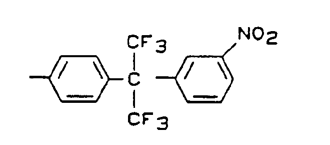 with the proviso that if Y represents the group -N=N-Z, X is only NO₂, and if Y represents the group -CH₃, X is only hydrogen.

2. A compound of the formula

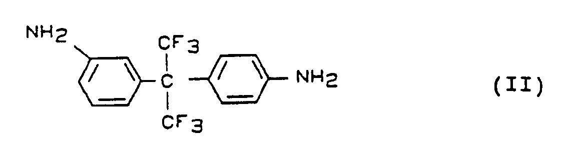

3. A process for the preparation of the compound of the formula (II) in a multi-stage process, which comprises
1a) subjecting 2-(4-methylphenyl)hexafluoropropan-2-ol to a condensation reaction with benzene or
1b) subjecting 2-phenylhexafluoropropan-2-ol to a condensation reaction with toluene in the presence of hydrogen fluoride,
2) oxidizing the resulting 2-(4-methylphenyl)-2-phenylhexafluoropropane (III) to give the corresponding carboxylic acid derivative (IV),
3) obtaining 2-(4-carboxyphenyl)-2-(3-nitrophenyl)hexafluoropropane (V) from (IV) by nitration and then
4) converting this into 2-(4-carboxamidophenyl)-2-(3-nitrophenyl)hexafluoropropane (VI) by reaction with amidosulfonic acid,
5) forming 4,4′-bis[2-(3-nitrophenyl)hexafluoroisopropyl]azobenzene (VII) from (VI) by reaction with an alkali metal hypohalide solution in an alkaline medium and then
6) reducing the product to give 2-(3-aminophenyl)-2-(4-aminophenyl)hexafluoropropane (II).

4. The process as claimed in claim 3, wherein a minor amount of 2-(3-nitrophenyl)-2-(4-aminophenyl)hexafluoropropane (VIIa) is formed in stage 5) in addition to the compound (VII).

5. The process as claimed in claim 3, wherein the reduction in stage 6) is carried out catalytically with a hydrogenation catalyst of a transition metal of the Periodic Table, in particular at least one metal of sub-group VIII, or by a stoichiometric method.

6. The process as claimed in any one of claims 3 to 5, wherein the compounds (V), (VI) and (VIIa) are reduced in accordance with stage 6) to give the corresponding amino compounds.

7. The process as claimed in claim 3, wherein the oxidation of the compound (III) is carried out by passing atmospheric oxygen into an acid organic medium at temperatures of 120 to 220°C under a pressure between 5 and 40 bar in the presence of a mixture of compounds of the metals Co and Mn and of bromide ions.

8. The process as claimed in claim 7, wherein the ratio of cobalt to manganese is 3:1 to 1:3, preferably 1:1, the sum of the concentrations of the two elements cobalt and manganese being 0.01-0.20 g atoms/kg of total reaction mass, preferably 0.02-0.12 and in particular 0.04-0.08 g atoms/kg.

9. The process as claimed in either of claims 7 and 8, wherein the catalyst contains cerium as an additional metal ion in a ratio of the sum of cobalt and manganese to cerium of 1:0.02-2, preferably 1:0.05-1 and in particular 1:0.2-0.6.

10. The process as claimed in any one of claims 7 to 9, wherein the metal ions are added in the form of acetate compounds.

11. The process as claimed in any one of claims 7 to 10, wherein the bromine is employed in the form of a bromide or as a solution of hydrogen bromide in water or glacial acetic acid.

12. The use of the compound as claimed in claim 2 for the preparation of a linear polycarboxylic acid amide or imide.

13. The use as claimed in claim 12, wherein shaped articles, films and fibers are produced.

## Revendications

1. Composés de formule : 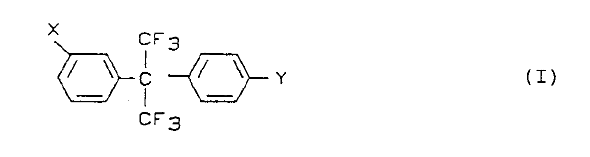 X désignant l'hydrogène ou le groupe NO₂ ou NH₂ et
Y un groupe CH₃, COOH, CONH₂, NH₂ ou -N=N-Z, Z étant le groupe 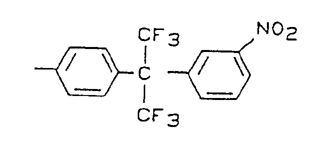 avec la condition que si Y est le groupe -N=N-Z, X ne puisse être que le groupe NO₂, et que si Y est le groupe -CH₃, X ne puisse être que l'hydrogène.

2. Le composé selon la revendication 1, de formule :

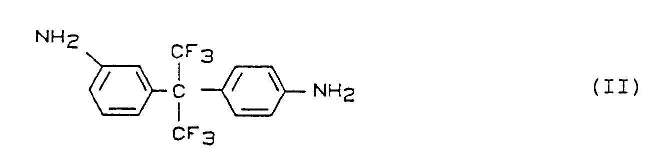

3. Procédé de préparation du composé de formule II selon la revendication 2 en plusieurs étapes, procédé caractérisé en ce que :
1a) on condense le 2-(4-méthylphényl)-hexafluoropropanol-2 avec du benzène, ou bien
1b) le 2-phénylhexafluoropropanol-2 avec du toluène, en présence de fluorure d'hydrogène,
2) on oxyde le 2-(4-méthylphényl)-2-phényl-hexafluoropropane (III) ainsi obtenu en acide carboxylique correspondant (IV),
3) en nitrant cet acide (IV) on obtient le 2-(4-carboxyphényl)-2-(3-nitrophényl)-hexafluoropropane (V)
4) que l'on transforme par réaction avec l'acide aminosulfonique en 2-(4-carboxylamidophényl)-2-(3-nitrophényl)-hexafluoropropane (VI),
5) en faisant réagir ce composé (VI) en milieu alcalin avec une solution d'un hypohalogénite de métal alcalin on forme le 4,4′-bis-[2-(3-nitrophényl)-hexafluoroisopropyl]-azobenzène (VII)
6) que l'on réduit ensuite en 2-(3-aminophényl)-2-(4-aminophényl)-hexafluoropropane (II).

4. Procédé selon la revendication 3, caractérisé en ce qu'il se forme dans l'étape 5), en plus du composé (VII), une moindre proportion de 2-(3-nitrophényl)-2-(4-aminophényl)-hexafluoropropane (composé VIIa).

5. Procédé selon la revendication 3, caractérisé en ce que l'on effectue la réduction de l'étape 6) par voie catalytique avec des catalyseurs d'hydrogénation de métaux de transition de la classification périodique des éléments, en particulier avec au moins un métal du sous-groupe VIII de cette classification, ou bien suivant des méthodes stoechiométriques.

6. Procédé selon une ou plusieurs des revendications 3 à 5, caractérisé en ce que l'on réduit dans l'étape 6) les composés (V), (VI) et (VIIa) en leurs composés aminés correspondants.

7. Procédé selon la revendication 3, caractérisé en ce que l'on effectue l'oxydation du composé (III) en faisant passer de l'oxygène dans un milieu organique acide à des températures de 120 à 220 °C et sous une pression de 5 à 40 bar, en présence d'un mélange de composés du cobalt et du manganèse ainsi que d'ions bromure.

8. Procédé selon la revendication 7, caractérisé en ce que le rapport du cobalt au manganèse est de 3:1 à 1:3, de préférence de 1:1, la somme des concentrations des deux éléments cobalt et manganèse étant de 0,01-0,20 atome-g par kg de la masse réactionnelle totale, de préférence de 0,02-0,12 et en particulier de 0,04-0,08 atome-g par kg.

9. Procédé selon la revendication 7 ou 8, caractérisé en ce que le catalyseur comprend en outre des ions cérium dans un rapport de la somme du cobalt et du manganèse au cérium tel que 1:0,02-2, de préférence de 1:0,05-1 et en particulier de 1:0,2-0,6.

10. Procédé selon une ou plusieurs des revendications 7 à 9, caractérisé en ce que les ions de métaux sont ajoutés sous la forme d'acétates.

11. Procédé selon une ou plusieurs des revendications 7 à 10, caractérisé en ce que l'on ajoute le brome à l'état de bromures ou bien en une solution de bromure d'hydrogène dans de l'eau ou de l'acide acétique cristallisable.

12. L'emploi du composé de la revendication 2 pour fabriquer des amides et imides d'acides polycarboxyliques linéaires.

13. Emploi selon la revendication 12, caractérisé en ce que l'on fabrique des objets moulés, des pellicules ou des fibres.
